# FASCICULE DE BREVET EUROPEEN

(11) **EP 0 971 972 B1**
(45) Date de publication et mention de la délivrance du brevet: **14.04.2004**
(21) Numéro de dépôt: 98917248.1
(22) Date de dépôt: 26.03.1998
(51) Int. Cl.: C08G 83/00, A61K 7/48

(54) **POLYMERES A FONCTION TERMINALE THIOL**
THIOL-ENDGRUPPEN ENTHALTENDE POLYMERISATE
POLYMERS WITH THIOL TERMINAL FUNCTION

(30) Priorité: 03.04.1997 FR 9704085
(43) Date de publication de la demande: 19.01.2000
(73) Titulaire: L'OREAL, 75008 Paris (FR)
(72) Inventeur: MAIGNAN, Jean, F-93290 Tremblay en France (FR)
(74) Mandataire: Dodin, Catherine
(86) Numéro de dépôt international: PCT/FR1998/000620
(87) Numéro de publication internationale: WO 1998/044024

(56) Documents cités:
- EP-A- 0 247 629
- EP-A- 0 556 871
- EP-A- 0 684 044
- WO-A-95/02397
- WO-A-95/34595

## Description

L'invention a pour objet de nouveaux dendrimères et polymères hyperbranchés comportant des groupes fonctionnels thiols, leur procédé de préparation, l'utilisation de ces dendrimères et/ou de ces polymères hyperbranchés comme agents anti-oxydants.

De nombreux polymères hyperbranchés et dendrimères ont déjà été décrits. On peut se reporter par exemple à : D. A. Tomalia et al, Angew. Chem. Int. Engl. 29 (1990) 138-175 ; N. Ardoin et D. Astruc, Bull. Soc. Chim. Fr. (1995) 132, 875-909 ; B. I. Voit, Acta Polymer, 46, 87-99 (1995).

La possibilité de préparer des dendrimères comportant des groupes terminaux thiols a été envisagée par certains auteurs, comme par exemple D. A. Tomalia dans US-4,587,329 et EP-A-234408 sans que cette préparation soit jamais effectivement réalisée, ni que les propriétés étonnantes de ces molécules, propriétés qui ont été mises en évidence par la demanderesse, soient mentionnées ou suggérées dans l'art antérieur.

En outre, le procédé de préparation de polymères hyperbranchés et de dendrimères à groupes fonctionnels thiols est nouveau et il présente de nombreux avantages parmi lesquels le fait d'avoir un bon rendement de synthèse, d'utiliser des produits de départ disponibles commercialement, d'être simple à mettre en oeuvre.

Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications ; une couche de chaînes terminales.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%. On peut faire réagir un groupement terminal T sur le polymère hyperbranché pour obtenir une fonctionnalité particulière en extrémité de chaînes.

De tels polymères sont décrits en particulier dans B.I.Voit, Acta Polymer., 46, 87-99 (1995) ; EP-682059 ; WO-9614346 ; WO-9614345 ; WO-9612754.

Plusieurs polymères hyperbranchés peuvent être associés entre eux, par une liaison covalente ou un autre type de liaison, par l'intermédiaire de leurs groupes terminaux. De tels polymères dits pontés ou « bridged » entrent dans la définition des polymères hyperbranchés selon la présente invention.

Les dendrimères sont des polymères et oligomères hautement ramifiés, également connus, ayant une structure chimique bien définie et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles , qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la N^{ième} génération sont les groupes fonctionnels terminaux des fuseaux de la N^{ième} génération ou génération terminale. De tels polymères sont décrits en particulier dans D.A.Tomalia, A.M.Naylor et W.A.Goddard III, *Angewandte Chemie*, Int.Ed.Engl.29, 138-175 (1990); C.J.Hawker et J.M.J.Frechet, *J.Am.Chem.Soc*., 112, 7638 (1990) ; B.I.Voit, Acta Polymer., 46, 87-99 (1995) ; N.Ardoin et D.Astruc, Bull. Soc. Chim. Fr. 132, 875-909 (1995).

On peut également plus particulièrement définir les dendrimères par la formule (DI) suivante :
C[A₁B₁(A₂B₂(...(Aₙ₋₁Bₙ₋₁(AₙBₙ(T)rₙ)rₙ₋₁)rₙ₋₂...)r₂)r₁]s (DI)
dans laquelle :
- C représente le coeur, relié par un nombre s de fonctionnalités à s fuseaux A₁B₁ par l'intermédiaire des groupements A₁ ;
- s est un nombre entier supérieur ou égal à 1 et inférieur ou égal au nombre de fonctionnalités de C ;
- pour chaque fuseau (AᵢBᵢ) (i=1, 2.....n), le groupement Bᵢ est relié à rᵢ groupements Aᵢ₊₁ d'un fuseau (Aᵢ₊₁Bᵢ₊₁) ;
- chaque groupement Aᵢ (i ≥ 2) est relié à un seul groupement Bᵢ₋₁ du fuseau (Aᵢ₋₁Bᵢ₋₁) ;
- rᵢ (i=1, 2.....n-1) représente le nombre de fonctionnalités du groupement Bᵢ appartenant au fuseau (AᵢBᵢ) ; ri étant un nombre entier supérieur ou égal à 2
- l'indice i (i=1, 2.....n) est un nombre entier qui désigne la génération de chaque fuseau ;
- le fuseau de n^{ième} génération AₙBₙ est chimiquement lié à un nombre rₙ de groupes terminaux T ; rₙ étant un entier supérieur ou égal à zéro.
   La définition des dendrimères donnée ci-dessus inclut des molécules à ramifications symétriques ; elle inclut également des molécules à ramification-non symétrique, comme par exemple les dendrimères dont les fuseaux sont des groupements lysine,
dans lesquels le branchement d'une génération de fuseaux sur la précédente se fait sur les amines α et ε de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

L'invention a pour objet une composition caractérisée par le fait qu'elle comprend, dans un support cosmétiquement ou dermatologiquement acceptable, au moins un polymère choisi parmi les polymères hyperbranchés et les dendrimères, comportant des groupements fonctionnels répondant à la formule (I) : 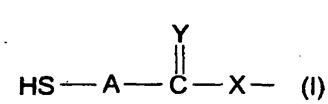 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N; ledit groupement alcane di-yle étant substitué par une fonction choisie parmi :
   - amino : -NH₂, éventuellement sous forme d'un sel d'un acide minéral ou organique,
   - acylamino : -NH-COR, dans lequel R représente un groupement alkyle en C₁-C₁₀ linéaire, ramifié ou cyclique, saturé ou insaturé,
   - acide carboxylique,
   - ester en C₁-C₁₀.

Un autre objet de l'invention est une composition caractérisée par le fait qu'elle comprend, dans un support cosmétiquement ou dermatologiquement acceptable, au moins un polymère choisi parmi les polymères hyperbranchés et les dendrimères, comportant des groupements fonctionnels répondant à la formule (I) : 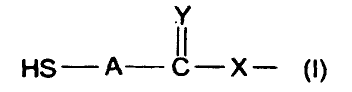 dans laquelle:
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N ; éventuellement substitué par une fonction choisie parmi :
   - amino : -NH₂, éventuellement sous forme d'un sel d'un acide minéral ou organique,
   - acylamino : -NH-COR, dans lequel R représente un groupement alkyle en C₁-C₁₀ linéaire, ramifié ou cyclique, saturé ou insaturé,
   - acide carboxylique,
   - ester en C₁-C₁₀.
associé à un composé choisi parmi l'acide thioglycolique, l'acide thiolactique et la cystéine.

Un autre objet de l'invention est l'utilisation d'une composition comprenant, dans un support cosmétiquement ou dermatologiquement acceptable, au moins un polymère choisi parmi les polymères hyperbranchés et les dendrimères, comportant des groupements fonctionnels répondant à la formule (I) : 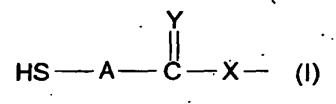 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N ; éventuellement substitué par une fonction choisie parmi :
   - amino : -NH₂, éventuellement sous forme d'un sel d'un acide minéral ou organique,
   - acylamino : -NH-COR, dans lequel R représente un groupement alkyle en C₁-C₁₀ linéaire, ramifié ou cyclique, saturé ou insaturé,
   - acide carboxylique,
   - ester en C₁-C₁₀,
   pour le traitement des ongles et des cheveux.

Un autre objet de l'invention est l'utilisation de ces polymères comme réducteur, comme agent anti-oxydant ou bien, appliqué sur un support, pour former un film résultant de la formation de ponts disulfures intermoléculaires conduisant ainsi à un polymère dudit polymère hyperbranché ou dendrimère.

Un autre objet de l'invention est un polymère choisi parmi les polymères hyperbranchés et les dendrimères caractérisé par le fait qu'il comporte des groupements fonctionnels répondant à la formule (I) : 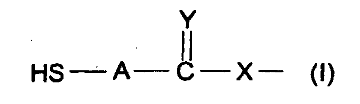 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé, éventuellement interrompu par un ou plusieurs hétéroatomes, comme O ou N, ledit groupement alcane di-yle étant substitué par une fonction choisie parmi :
   - amino (-NH₂), éventuellement sous forme d'un sel d'un acide minéral ou organique,
   - acylamino (-NH-COR), dans lequel R représente un groupement alkyle en C₁-C₁₀ linéaire, ramifié ou cyclique, saturé ou insaturé,
   - acide carboxylique,
   - ester en C₁-C₁₀.
De préférence, Y = O.

X représente un groupement nucléophile, préférentiellement :
un atome d'oxygène
   ou
un groupement -NR'- dans lequel R' est choisi parmi un atome d'hydrogène ; un groupement alkyle en C₁-C₆, linéaire ou ramifié, saturé ou insaturé ; un groupement mono- ou poly-hydroxyalkyle en C₁-C₆, linéaire ou ramifié, saturé ou insaturé ; un groupement aminoalkyle en C₁-C₆ ou un groupement polyalkylèneimine.

Par exemple, A peut être un groupement méthylène, éthylène, propylène, méthylpropylène, éthylpropylène, tétraméthylène, pentaméthylène, hexaméthylène, phénylène, phényl di-yle etc.....

Avantageusement, A représente un radical répondant à l'une des formules (a) à (d) suivantes :

-CHR¹-CHR²-CHR³- (a)

- CHR'¹-CHR'²-CHR'³-CHR'⁴- (b)

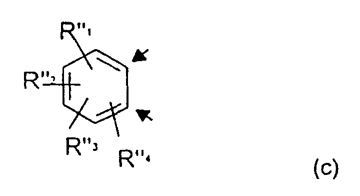

-(CHR'''¹)ₖ-(CHR'''²)-CH(CO₂H)-NH- (d)

dans lesquelles R¹, R², R³, R'¹, R'², R'³ et R'⁴, R'''¹, R'''², identiques ou différents représentent : l'atome d'hydrogène, un radical alkyle en C₁-C₆, linéaire, ramifié ou cyclique, saturé ou insaturé, un radical amino -NH₂, un radical acide carboxylique -COOH , un radical alkyl amino en C₁-C₁₀; R"¹, R"², R"³ et R"⁴, identiques ou différents représentent : l'atome d'hydrogène, un radical alkyle en C₁-C₄, linéaire ou ramifié, saturé ou insaturé, les flèches indiquant les positions des substitutions dans la formule (c), k est un entier, préférentiellement k=0 ou 1.

Préférentiellement A est choisi parmi :

- CH₂-CH(CO₂H)-NH- et Y=O

-(CH₂)₂-(CH₃CONH)CH- et Y=O

-(CH₂)₃- et Y=O ou Y=NH

De façon avantageuse, A est le radical triméthylène -CH₂-CH₂-CH₂-, et Y=O, le composé selon l'invention répondant alors à la formule (II) suivante : 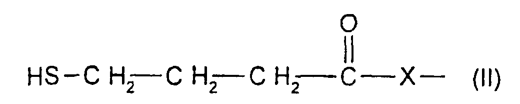 dans laquelle :
X représente un groupement nucléophile, préférentiellement :
   un atome d'oxygène
      ou
   un groupement -NR'- dans lequel R' est choisi parmi un atome d'hydrogène ; un groupement alkyle en C₁-C₆, linéaire ou ramifié, saturé ou insaturé ; un groupement mono- ou poly-hydroxyalkyle en C₁-C₆, linéaire ou ramifié, saturé ou insaturé; un groupement aminoalkyle en C₁-C₆ ou un groupement polyalkylèneimine.

Préférentiellement, selon l'invention, X est choisi parmi : l'atome d'oxygène et un groupement NH.

Dans le cas des dendrimères, le groupement nucléophile X est généralement un groupement fonctionnel terminal. Dans le cas des polymères hyperbranchés, comme par exemple la polyéthylèneimine, le groupement X peut être une amine secondaire se trouvant sur l'une des branches du polymère sans être en position terminale.

Avantageusement selon l'invention, au moins 10%, en nombre, des groupements X du polymère hyperbranché ou du dendrimère sont greffés par un groupement fonctionnel : 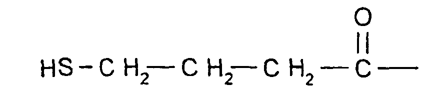 et encore plus préférentiellement au moins 40%. Le pourcentage de groupes fonctionnels thiols par rapport au nombre total de groupes fonctionnels X du polymère hyperbranché ou du dendrimère qui sont susceptibles d'être substitués par un groupement : 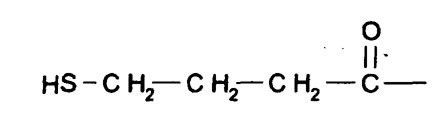 est adapté en fonction des autres caractéristiques du polymère hyperbranché ou du dendrimère, notamment le nombre de générations, la nature des fuseaux, en fonction des propriétés attendues, en particulier la solubilité du polymère hyperbranché ou du dendrimère. De telles adaptations sont à la portée de l'homme du métier par de simples essais.

Un procédé de préparation de polymères hyperbranchés et de dendrimères à groupes terminaux thiols, est caractérisé par le fait que l'on fait réagir un polymère de départ, choisi parmi les polymères hyperbranchés et les dendrimères dont les chaînes ou les groupes terminaux comportent une fonction nucléophile, avec une thio-lactone ou un imino-thiolane, selon le schéma réactionnel suivant : 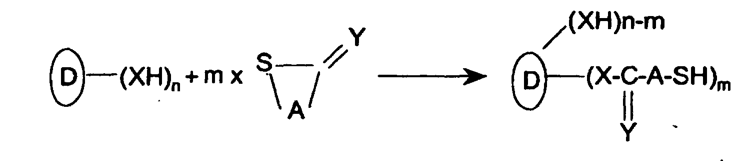 dans lequel 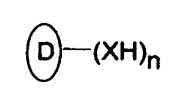 représente un dendrimère ou un polymère hyperbranché comportant n fonctions XH, telles que définies ci-dessus , m est un entier m ≤ n et A représente un radical répondant à l'une des formules (a) à (d) suivantes :

-CHR¹-CHR²-CHR³- (a)

-CHR'¹-CHR'²-CHR'³-CHR'⁴- (b)

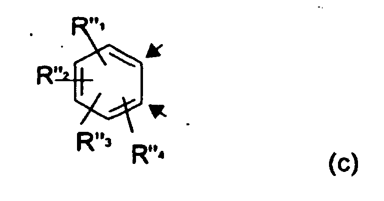

-(CHR'''¹)ₖ-(CHR'''²)-CH(CO₂H)-NH- (d)

dans lesquelles R¹, R², R³, R'¹, R'², R'³ et R'⁴, R'''¹, R'''², identiques ou différents représentent : l'atome d'hydrogène, un radical alkyle en C₁-C₆, linéaire ou ramifié, saturé ou insaturé, un radical amino -NH₂, un radical acide carboxylique -COOH , un radical alkyl amino en C₁-C₁₀, R"¹, R"², R"³ et R"⁴, identiques ou différents représentent : l'atome d'hydrogène, un radical alkyle en C₁-C₄, linéaire ou ramifié, saturé ou insaturé, les flèches indiquant les positions des substitutions dans la formulé (c) ; k est un entier, préférentiellement k=0 ou 1.

De préférence, dans le procédé selon l'invention, le composé : 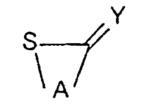 est choisi parmi :
l'acide 2-oxo-4-thiazolidine carboxylique également connu sous le nom de procystéine : 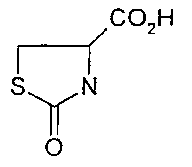 la N-acétyl homocystéine-thiolactone : 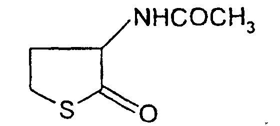 la γ-thiobutyrolactone : 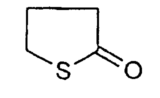 l'imino thiolane :

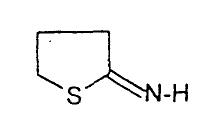

Avantageusement, dans le procédé selon l'invention, on utilise un réactif choisi parmi la procystéine, la N-acétyl homocystéine-thiolactone et la γ-thiobutyrolactone.

Avantageusement, dans le procédé selon l'invention, le composé : 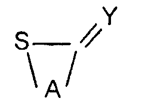 est la γ-thiobutyrolactone.

La γ-thiobutyrolactone est un produit commercial.

La réaction d'ouverture de la thio-lactone ou de l'imino-thiolane est généralement conduite sous atmosphère inerte soit dans l'eau, soit dans un solvant aromatique tel que le toluène ou un alcool. tel que le méthanol, l'éthanol, l'isopropanol ou le butanol, et, suivant le point d'ébullition du solvant, à une température comprise entre 0°C et 110° C.

Toutefois, selon une forme préférée de l'invention la réaction est conduite dans l'eau et dans ce cas on mélange dans des proportions stoechiométriques (par rapport aux fonctions -XH du polymère) le dendrimère ou le polymère hyperbranché et la thio-lactone ou l'imino-thiolane, puis on porte le mélange, sous atmosphère inerte, à une température comprise entre 0°C et 110° C.

Lorsque l'on souhaite conserver des fonctions amines ou hydroxyles libres parmi les n groupements fonctionnels du polymère hyperbranché ou du dendrimère, on met en oeuvre la quantité voulue, m, de molécules de thio-lactone ou d'imino-thiolane par molécule de dendrimère ou de polymère hyperbranché, pour obtenir le dendrimère ou le polymère hyperbranché ayant n-m fonctions amines ou hydroxyles libres et m fonctions thiols.

Suivant la basicité du dendrimère ou du polymère hyperbranché , le temps de réaction peut être compris entre 1 et 48 heures, l'évolution de la réaction étant suivie en dosant l'apparition du thiol et/ou la disparition de la fonction amine ou de la fonction alcool. Il est également possible de suivre la transformation du polymère de départ par électrophorèse.

Avantageusement, lorsque le radical -A- comporte une fonction acide libre ou uné fonction amine libre, on neutralise cette fonction avant introduction du polymère de départ dans le milieu réactionnel.

Si au cours de la réaction une certaine quantité de thiol est oxydée en disulfure correspondant, le mélange réactionnel est alors dilué par deux fois son volume d'eau et agité en milieu acide en présence de poudre de zinc pendant 1 à 3 heures. La majorité du disulfure étant réduite, le mélange est alors filtré et on obtient une solution du composé attendu qui peut être utilisée directement.

Les dendrimères et les polymères hyperbranchés à fonction mercapto-alkylamide peuvent également être obtenus de façon connue par réaction d'amidification de l'acide mercapto-alcanoïque correspondant ou de ses esters. On peut représenter un tel procédé par le schéma réactionnel suivant : 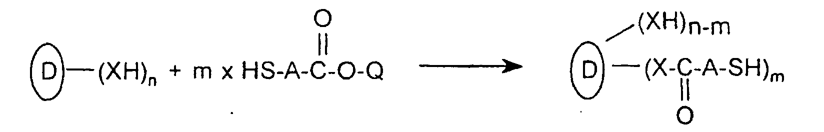 dans lequel X, n, m, A, 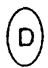 , ont la même signification que ci-dessus et Q représente l'atome d'hydrogène ou un radical alkyle en C₁-C₁₀, saturé ou insaturé, linéaire ou ramifié.

Cependant, le procédé de préparation décrit ci-dessus à partir d'une thio-lactone ou d'un imino-thiolane est plus rapide et n'entraîne pas la formation de produits secondaires qui parfois sont très malodorants et contaminent le produit final.

On peut se reporter aux documents suivants dans lesquels sont décrits des polymères hyperbranchés et des dendrimères dont le groupe terminal comporte une fonction amine : US-4,694,064 ; US-4,507,466 ; US-4,631,337 ; US-4,558,120 ; US-4,568,737 ; US-4,587,329 ; WO-A-9502008 ; WO-A-9314147 ; EP-234408 ; US-4,289,872 ; US-4,360,646 ; Proc. Natl. Acad. Sci. USA, 85, 5409-5413 (1988) ; WO95/02008 ; WO93/14147 ; J. Am. Chem. Soc. 117, 9764 (1195) ; FR-2734268.

Les dendrimères à groupes terminaux aminés sont par exemple dés polyamidoamines, comme ceux commercialisés sous la marque STARBURST PAMAM par la société DENDRITECH (copolymères séquencés d'éthylène diamine et d'acrylate de méthyle). Ils peuvent également être choisis parmi les dendrimères du type polyalkylène polyamine, comme par exemple les polyéthylèneimines et les polypropylèneimines fabriqués par la société DSM et décrits dans les brevets : WO93/14147 et WO95/02008. Ils peuvent également appartenir à la famille des polylysines telles que décrites dans le brevet US-4,360,646. Parmi les polymères hyperbranchés on peut citer les polyalkylène polyamines comme la polyéthylèneimine commercialisée par la société BASF sous les noms de marque POLYMIN et LUPASOL.

Les polymères hyperbranchés et les dendrimères à groupes fonctionnels aminés peuvent également être constitués d'un coeur et de générations d'unités de base, monomères ou fuseaux, de toutes natures, sur lesquels un groupe terminal T porteur d'une fonction amine a été greffé.

Des polymères hyperbranchés et des dendrimères à groupes terminaux hydroxylés, en particulier des polymères de la famille des polyesters, sont décrits dans les documents suivants: US 4,587,329 ; WO 93/17060 ; WO 92/14543 ; J. Am. Chem. Soc., 113, 4583-4588 (1991) ; Macromolecules 25, 4583-4587 (1992) ; US 5,196,502 ; US 5,214,122.

De préférence, le polymère de départ est choisi parmi les dendrimères.

A l'issue de la réaction, la nature du coeur et des branches du polymère de départ ne sont pas modifiées. Eventuellement, en fonction des proportions de réactifs utilisées, une partie des groupements terminaux de départ ne sont pas modifiés. Ainsi si l'on part d'une polyamidoamine, que l'on traite par la γ-thiobutyrolactone, on obtient par le procédé de l'invention un polyamide à fonctions terminales mercapto-4-butyramide. Si l'on part d'une polyalkylène polyamine, on obtient par le procédé de l'invention une polyalkylène polyamine greffée par des groupements fonctionnels mercapto-4-butyramide. Si l'on part d'un polyester, on obtient par le procédé de l'invention un polyester à fonctions terminales mercapto-4-butyramide.

Ces nouveaux polymères ont des propriétés réductrices et, peuvent être utilisés à la place des réducteurs classiquement utilisés, par exemple dans des applications cosmétiques, comme par exemple le traitement des ongles et des cheveux. En particulier, ces nouveaux polymères peuvent être utilisés comme réducteurs dans des compositions de permanente.

Les polymères selon l'invention comportant une fonction terminale -SH, ils peuvent être utilisés comme conservateurs pour protéger des produits particulièrement sensibles à l'oxydation. Ils peuvent être utilisés comme agent anti-oxydant dans des compositions de toute nature, notamment dans des compositions cosmétiques ou pharmaceutiques, par exemple dans des compositions capillaires, comme des shampooings, des lotions, des gels, des émulsions, des laques pour cheveux, des compositions à rincer, à appliquer avant ou après shampooing, avant ou après coloration ou décoloration, avant, pendant ou après permanente ou défrisage, des lotions ou un gel coiffants ou traitants, des lotions ou des gels pour le brushing ou la mise en plis, des compositions de permanente ou de défrisage, de coloration ou décoloration des cheveux. Ils peuvent également être utilisés comme agents anti-oxydants dans des produits de soin de la peau ou de maquillage comme les produits de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", vernis à ongles, dans les lotions, laits et crèmes de soin ou de nettoyage de la peau.

L'invention a en outre pour objet une composition comprenant dans un support cosmétiquement ou dermatologiquement acceptable au moins un polymère selon l'invention.

Appliqués sur un support, les polymères selon l'invention peuvent former un film résultant de la formation de ponts disulfures intermoléculaires conduisant ainsi à un polymère du dendrimère ou du polymère hyperbranché à fonction terminale -SH.

Ce film peut se former à partir de quelques fonctions thiols. Les autres fonctions restant libres et étant susceptibles d'avoir une action réductrice.

L'utilisation des polymères selon l'invention comme agent anti-oxydant peut être envisagée directement en solution dans le milieu à protéger de l'oxydation, comme c'est habituel avec les anti-oxydants classiques que sont l'acide thioglycolique, l'acide thiolactique ou la cystéine.

On peut également envisager, si le polymère est insoluble dans le milieu à protéger de l'oxydation, de le fixer, par exemple, sous forme de film ou de pastille, aux parois ou dans le bouchon du flacon contenant la formulation à protéger.

On peut également, dans ce second cas, associer au dendrimère ou au polymère hyperbranché selon l'invention une quantité faible d'un des mercaptans habituellement utilisés comme anti-oxydant, comme par exemple l'acide thioglycolique, l'acide thiolactique ou la cystéine. Cette association permet de limiter ainsi la quantité de thiol soluble dans la composition à protéger et donc les inconvénients habituels qui lui sont associés : odeur, modification de la couleur, tout en conservant une efficacité remarquable. L'invention a donc également pour objet une telle association.

Les polymères hyperbranchés et dendrimères selon l'invention présentent en particulier l'avantage d'être beaucoup moins odorants que les thiols habituellement utilisés comme anti-oxydants : à température ambiante, ils sont quasiment inodores.

Du fait de leur structure particulière, les polymères hyperbranchés et dendrimères selon l'invention pénètrent peu dans la kératine ou dans l'épiderme, ils sont donc peu sensibilisants et ne posent pas de problèmes de toxicité.

Des exemples non limitatifs vont être donnés ci-après à titre d'illustration.

### EXEMPLES

### Exemple 1 : Dendrimère à coeur éthylène diamine, à fuseau éthylène diamine et acrylate de méthyle de génération 1 possédant 8 fonctions SH en surface

A 2 grammes d'une solution aqueuse à 55.7g/100g de dendrimère commercialisé par la société Dendritech sous le nom PAMAM Starburst à coeur éthylène diamine de génération 1 (8 fonctions NH₂ en surface) dilués par 2 ml d'eau, on ajoute 540µl de γ-thiobutyrolactone (soit 1 équivalent calculés par rapport à l'ensemble des fonctions amines primaires) sous atmosphère inerte à température ambiante. Le milieu, initialement hétérogène, devient rapidement homogène (1 heure). Après 48 heures sous agitation, on ne détecte que des traces de γ-thiobutyrolactone dans le milieu. On lave 3 fois par 10 ml d'éther diéthylique, puis on fait barboter de l'azote dans la phase aqueuse ainsi obtenue pour éliminer toute trace d'éther.
La solution aqueuse ainsi obtenue est analysée par RMN. On constate que toutes les fonctions amines primaires initiales sont sous forme -NH-CO-(CH₂)₃-SH.
La teneur en matière active de cette phase aqueuse est de 37.71g/100g. Le dendrimère ainsi obtenu est utilisé tel quel en solution aqueuse.
Masse molaire du produit synthétisé: 2247 g.mol⁻¹
Formule Brute: C₉₄ H₁₇₆ N₂₆ O₂₀ S₈

### Exemple 2 : Dendrimère à coeur éthylène diamine, à fuseau éthylène diamine et acrylate de méthyle de génération 3 possédant 16 fonctions NH₂ et 16 fonctions SH en surface

A 4 grammes d'une solution aqueuse à 47.85g/100g de dendrimère commercialisé par la société Dendritech sous le nom Starburst PAMAM, à coeur éthylène diamine, de génération 3 (32 fonctions NH₂ en surface) dilués par 4 ml d'eau, on ajoute 384 µl de γ-thiobutyrolactone (soit 0.5 équivalent calculés par rapport à l'ensemble des fonctions amines primaires) sous atmosphère inerte à température ambiante. Le milieu hétérogène à l'ajout devient rapidement homogène. Après 20 heures sous agitation, on ne détecte plus de γ-thiobutyrolactone dans le milieu.

La solution aqueuse ainsi obtenue est analysée par RMN. On constate que 50% des fonctions amines primaires initiales sont sous forme -NH-CO-(CH₂)₃-SH.
La teneur en matière active de cette phase aqueuse est de 27.75g/100g. Le dendrimère ainsi obtenu est utilisé tel quel en solution aqueuse.
Masse molaire du produit synthétisé: 8532 g.mol⁻¹
Formule Brute: C₃₆₆ H₇₀₄ N₁₂₂ O₇₆ S₁₆

### Exemple 3 : Dendrimère à coeur éthylène diamine, à fuseau éthylène diamine et acrylate de méthyle de génération 1 possédant 1.6 fonctions NH₂ et 6.4 fonctions SH en surface

A 2 grammes d'une solution aqueuse à 55.7g/100g de dendrimère commercialisé par la société Dendritech sous le nom Starburst PAMAM à coeur éthylène diamine de génération 1 (8 fonctions NH₂ en surface) dilués par 2 ml d'eau, on ajoute 431µl de γ-thiobutyrolactone (soit 0.8 équivalent calculés par rapport à l'ensemble des fonctions amines primaires) sous atmosphère inerte à température ambiante. Le milieu, initialement hétérogène, devient rapidement homogène (1 heure). Après 24 heures sous agitation, on ne détecte plus de γ-thiobutyrolactone dans le milieu.
La solution aqueuse ainsi obtenue est analysée par RMN. On constate que 80% des fonctions amines primaires initiales sont sous forme -NH-CO-(CH₂)₃-SH.
La teneur en matière active de cette phase aqueuse est de 35.99g/100g. Le dendrimère ainsi obtenu est utilisé tel quel en solution aqueuse.
Masse molaire du produit synthétisé: 2080.8 g.mol⁻¹
Formule Brute: C_{87.6} H_{166.4} N₂₆ O_{18.4} S_{6.4}

### Exemple 4 : Polymère branché polyéthylèneimine de poids moléculaire moyen PM=1200 possédant 6,75 fonctions SH

On dilue 1 gramme de polyéthylèneimine de poids moléculaire moyen PM=1200 commercialisée par la société POLYSCIENCES dans 3 grammes d'eau puis on ajoute à température ambiante 482µl de γ-thiobutyrolactone (soit 6,75 équivalents molaires calculés par rapport au poids moléculaire moyen du polymère) sous atmosphère inerte à température ambiante. Le milieu, initialement hétérogène, devient rapidement homogène (environ 30 minutes). Après 2 heures sous agitation, on ne détecte plus de γ-thiobutyrolactone dans le milieu. La phase aqueuse donne une réaction positive après révélation par le nitroprussiate de sodium. On constate ainsi que certaines des fonctions amines primaires initiales sont sous forme de -NH-CO-(CH₂)₃-SH.

La teneur en matière active de cette phase aqueuse est de 34.35g/100g. Le dendrimère ainsi obtenu est utilisé tel quel en solution aqueuse.

Masse molaire du produit synthétisé: 1889.6 g.mol⁻¹

## Revendications

1. Composition **caractérisée par le fait qu'**elle comprend, dans un support cosmétiquement ou dermatologiquement acceptable, au moins un polymère choisi parmi les polymères hyperbranchés et les dendrimères, comportant des groupements fonctionnels répondant à la formule (I) : 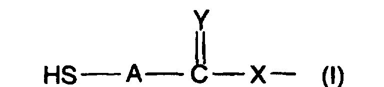 dans laquelle:
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N; ledit groupement alcane di-yle étant substitué par une fonction choisie parmi :
- amino : -NH₂, éventuellement sous forme d'un sel d'un acide minéral ou organique,
- acylamino : -NH-COR, dans lequel R représente un groupement alkyle en C₁-C₁₀ linéaire, ramifié ou cyclique, saturé ou insaturé,
- acide carboxylique,
- ester en C₁-C₁₀.

2. Composition **caractérisée par le fait qu'**elle comprend, dans un support cosmétiquement ou dermatologiquement acceptable, au moins un polymère choisi parmi les polymères hyperbranchés et les dendrimères, comportant des groupements fonctionnels répondant à la formule (I) : 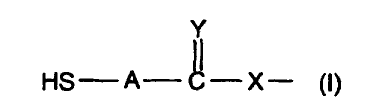 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O. ou N ; éventuellement substitué par une fonction choisie parmi :
- amino : -NH₂, éventuellement sous forme d'un sel d'un acide minéral ou organique,
- acylamino : -NH-COR, dans lequel R représente un groupement alkyle en C₁-C₁₀ linéaire, ramifié ou cyclique, saturé ou insaturé,
- acide carboxylique,
- ester en C₁-C₁₀.
associé à un composé choisi parmi l'acide thioglycolique, l'acide thiolactique et la cystéine.

3. Composition selon l'une des revendications précédentes, dans laquelle X représente un atome d'oxygène ou un groupement -NR'- dans lequel R' est choisi parmi un atome d'hydrogène ; un groupement alkyle en C₁-C₆, linéaire ou ramifié, saturé ou insaturé ; un groupement mono- ou poly-hydroxyalkyle en C₁-C₆, linéaire ou ramifié, saturé ou insaturé; un groupement aminoalkyle en C₁-C₆ ou un groupement polyalkylèneimine.

4. Composition selon l'une des revendications précédentes, dans laquelle le polymère appartient à la famille des polyamides, à la famille des polyalkylène polyamines, à la famille des polyesters ou à la famille des dendrimères.

5. Composition selon l'une des revendications précédentes, dans laquelle le polymère est une polyéthylèneimine.

6. Composition selon l'une des revendications précédentes, dans laquelle Y=O et A = -CH₂-CH(CO₂H)-NH- ou -(CH₂)₂-(CH₃CONH)CH- .

7. Composition selon la revendication 2, dans laquelle A = -(CH₂)₃- et Y=O ou NH.

8. Utilisation d'une composition comprenant, dans un support cosmétiquement ou dermatologiquement acceptable, au moins un polymère choisi parmi les polymères hyperbranchés et les dendrimères, comportant des groupements fonctionnels répondant à la formule (I) : 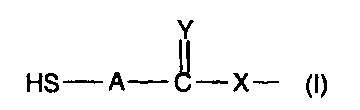 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N ; éventuellement substitué par une fonction choisie parmi :
- amino : -NH₂, éventuellement sous forme d'un sel d'un acide minéral ou organique,
- acylamino : -NH-COR, dans lequel R représente un groupement alkyle en C₁-C₁₀ linéaire, ramifié ou cyclique, saturé ou insaturé,
- acide carboxylique,
- ester en C₁-C₁₀,
pour le traitement des ongles et des cheveux.

9. Utilisation d'un polymère choisi parmi les polymères hyperbranchés et les dendrimères, comportant des groupements fonctionnels répondant à la formule (I) : 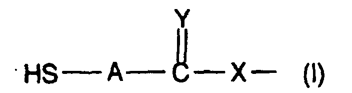 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N ; éventuellement substitué par une fonction
- amino : -NH₂, éventuellement sous forme d'un sel d'un acide minéral ou organique,
- acylamino : -NH-COR, dans lequel R représente un groupement alkyle en C₁-C₁₀ linéaire, ramifié ou cyclique, saturé ou insaturé,
- acide carboxylique,
- ester en C₁-C₁₀,
comme réducteur.

10. Utilisation selon la revendication 9, dans des compositions de permanente.

11. Utilisation d'un polymère choisi parmi les polymères hyperbranchés et les dendrimères, comportant des groupements fonctionnels répondant à la formule (I) : 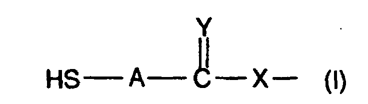 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N ; éventuellement substitué par une fonction
- amino : -NH₂, éventuellement sous forme d'un sel d'un acide minéral ou organique,
- acylamino : -NH-COR, dans lequel R représente un groupement alkyle en C₁-C₁₀ linéaire, ramifié ou cyclique, saturé ou insaturé,
- acide carboxylique,
- ester en C₁-C₁₀,
comme agent anti-oxydant.

12. Utilisation selon la revendication 11 dans des compositions capillaires, comme des shampooings, des lotions, des gels, des émulsions, des laques pour cheveux, des compositions à rincer, à appliquer avant ou après shampooing, avant ou après coloration ou décoloration, avant, pendant ou après permanente ou défrisage, des lotions ou un gels coiffants ou traitants, des lotions ou des gels pour le brushing ou la mise en plis, des compositions de permanente ou de défrisage, de coloration ou décoloration des cheveux; dans des produits de soin de la peau ou de maquillage comme les produits de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", vernis à ongles, dans les lotions, laits et crèmes de soin ou de nettoyage de la peau.

13. Utilisation d'un polymère choisi parmi les polymères hyperbranchés et les dendrimères, comportant des groupements fonctionnels répondant à la formule (I) : 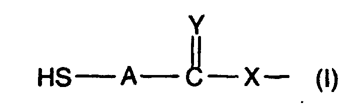 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé; éventuellement interrompu par un ou plusieurs hétéroatomes, comme O ou N ; éventuellement substitué par une fonction :
- amino : -NH₂, éventuellement sous forme d'un sel d'un acide minéral ou organique,
- acylamino : -NH-COR, dans lequel R représente un groupement alkyle en C₁-C₁₀ linéaire, ramifié ou cyclique, saturé ou insaturé,
- acide carboxylique,
- ester en C₁-C₁₀ ,
appliqué sur un support, pour former un film résultant de la formation de ponts disulfures intermoléculaires conduisant ainsi à un polymère dudit polymère hyperbranché ou dendrimère.

14. Utilisation selon l'une des revendications 8 à 13, dans laquelle :
A = -CH₂-CH(CO₂H)-NH- et Y=O, ou
A = -(CH₂)₂-(CH₃CONH)CH- et Y=O, ou
A = -(CH₂)₃- et Y=O, ou
A = -(CH₂)₃- et Y=NH.

15. Utilisation selon l'une des revendications 8 à 14, dans laquelle X représente un atome d'oxygène ou un groupement -NR'- dans lequel R' est choisi parmi un atome d'hydrogène ; un groupement alkyle en C₁-C₆, linéaire ou ramifié, saturé ou insaturé ; un groupement mono- ou poly-hydroxyalkyle en C₁-C₆, linéaire ou ramifié, saturé ou insaturé; un groupement aminoalkyle en C₁-C₆ ou un groupement polyalkylèneimine.

16. Utilisation selon l'une des revendications 8 à 15, dans laquelle le polymère appartient à la famille des polyamides, à la famille des polyalkylène polyamines, à la famille des polyesters ou à la famille des dendrimères.

17. Utilisation selon l'une des revendications 8 à 16, dans laquelle le polymère est une polyéthylèneimine.

18. Polymère choisi parmi les polymères hyperbranchés et les dendrimères **caractérisé par le fait qu'**il comporte des groupements fonctionnels répondant à la formule (I) : 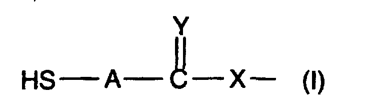 dans laquelle :
Y représente l'atome d'oxygène ou un groupement NH,
X représente un groupement nucléophile, et
A représente un groupement alcane di-yle en C₁-C₁₂, linéaire, ramifié ou cyclique, saturé, éventuellement interrompu par un ou plusieurs hétéroatomes, comme O ou N, ledit groupement alcane di-yle étant substitué par une fonction choisie parmi :
- amino (-NH₂), éventuellement sous forme d'un sel d'un acide minéral ou organique,
- acylamino (-NH-COR), dans lequel R représente un groupement alkyle en C₁-C₁₀ linéaire, ramifié ou cyclique, saturé ou insaturé,
- acide carboxylique,
- ester en C₁-C₁₀.

19. Polymère selon la revendication 18, **caractérisé par le fait que** :
A = -CH₂-CH(CO₂H)-NH- et Y=O, ou
A = -(CH₂)₂-(CH₃CONH)CH- et Y=O.

20. Polymère selon l'une quelconque des revendications 18 à 19, **caractérisé par le fait que** X représente un atome d'oxygène ou un groupement -NR'- dans lequel R' est choisi parmi un atome d'hydrogène; un groupement alkyle en C₁-C₆, linéaire ou ramifié, saturé ou insaturé; un groupement mono- ou poly-hydroxyalkyle en C₁-C₆, linéaire ou ramifié, saturé ou insaturé; un groupement aminoalkyle en C₁-C₆ ou un groupement polyalkylèneimine.

21. Polymère selon l'une quelconque des revendications 18 à 20, **caractérisé par le fait qu'**il appartient à la famille des polyamides, à la famille des polyalkylène polyamines, à la famille des polyesters ou à la famille des dendrimères.

22. Polymère selon la revendication 21, **caractérisé par le fait qu'**il est une polyéthylèneimine.

## Patentansprüche

1. Zusammensetzung, **dadurch gekennzeichnet, dass** sie in einem kosmetisch oder dermatologisch akzeptablen Träger mindestens ein Polymer enthält, das unter den hochverzweigten Polymeren und Dendrimeren ausgewählt ist, die funktionelle Gruppen der folgenden Formel (I) enthalten: 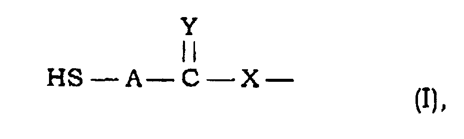 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino: -NH₂, gegebenenfalls in Form eines Salzes mit einer anorganischen oder organischen Säure,
- Acylamino: -NH-COR, wobei R eine geradkettige, verzweigte oder cyclische, gesättigte oder ungesättigte C₁₋₁₀-Alkylgruppe bedeutet,
- Carboxy,
- C₁₋₁₀-Ester.

2. Zusammensetzung, **dadurch gekennzeichnet, dass** sie in einem kosmetisch oder dermatologisch akzeptablen Träger mindestens ein Polymer, das unter den hochverzweigten Polymeren und Dendrimeren ausgewählt ist, die funktionelle Gruppen der Formel (I) enthalten: 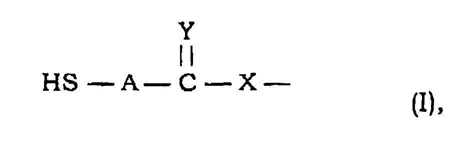 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino: -NH₂, gegebenenfalls in Form eines Salzes mit einer anorganischen oder organischen Säure,
- Acylamino: -NH-COR, wobei R eine geradkettige, verzweigte oder cyclische, gesättigte oder ungesättigte C₁₋₁₀-Alkylgruppe bedeutet,
- Carboxy,
- C₁₋₁₀-Ester,
in Kombination mit einer Verbindung enthält, die unter Thioglykolsäure, Thiomilchsäure und Cystein ausgewählt ist.

3. Zusammensetzung nach einem der vorhergehenden Ansprüche, wobei X ein Sauerstoffatom oder eine Gruppe -NR'- bedeutet, worin R' unter einem Wasserstoffatom; einer geradkettigen oder verzweigten, gesättigten oder ungesättigten C₁₋₆-Alkylgruppe; einer geradkettigen oder verzweigten, gesättigten oder ungesättigten Mono- oder Polyhydroxyalkylgruppe mit 1 bis 6 Kohlenstoffatomen; einer C₁₋₆-Aminoalkylgruppe oder einer Polyalkylenimingruppe ausgewählt ist.

4. Zusammensetzung nach einem der vorhergehenden Ansprüche, wobei das Polymer aus der Gruppe der Polyamide, aus der Gruppe der Polyalkylenpolyamine, aus der Gruppe der Polyester oder aus der Gruppe der Dendrimere stammt.

5. Zusammensetzung nach einem der vorhergehenden Ansprüche, wobei das Polymer ein Polyethylenimin ist.

6. Zusammensetzung nach einem der vorhergehenden Ansprüche, wobei Y = O und A = -CH₂-CH(CO₂H)-NH- oder -(CH₂)₂-(CH₃CONH)CH-.

7. Zusammensetzung nach Anspruch 2, wobei A = -(CH₂)₃- und Y = O oder NH.

8. Verwendung einer Zusammensetzung, die in einem kosmetisch oder dermatologisch akzeptablen Träger mindestens ein Polymer enthält, das unter den hochverzweigten Polymeren oder Dendrimeren ausgewählt ist, die funktionelle Gruppen der Formel (I) enthalten: 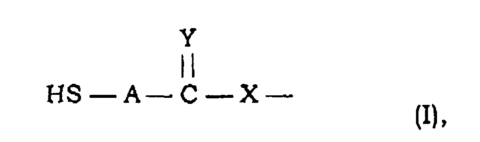 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino: -NH₂, gegebenenfalls in Form eines Salzes mit einer anorganischen oder organischen Säure,
- Acylamino: -NH-COR, wobei R eine geradkettige, verzweigte oder cyclische, gesättigte oder ungesättigte C₁₋₁₀-Alkylgruppe bedeutet,
- Carboxy,
- C₁₋₁₀-Ester
für die Behandlung der Nägel und der Haare.

9. Verwendung eines Polymers, das unter den hochverzweigten Polymeren und Dendrimeren ausgewählt ist, die funktionelle Gruppen der Formel (I) enthalten: 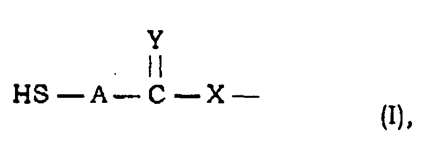 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino: -NH₂, gegebenenfalls in Form eines Salzes mit einer anorganischen oder organischen Säure,
- Acylamino: -NH-COR, wobei R eine geradkettige, verzweigte oder cyclische, gesättigte oder ungesättigte C₁₋₁₀-Alkylgruppe bedeutet,
- Carboxy,
- C₁₋₁₀-Ester,
als Reduktionsmittel.

10. Verwendung nach Anspruch 9 in Zusammensetzungen für permanente Verformungen.

11. Verwendung eines Polymers, das unter den hochverzweigten Polymeren und Dendrimeren ausgewählt ist, die funktionelle Gruppen der Formel (I) enthalten: 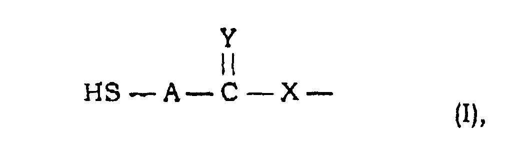 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino: -NH₂, gegebenenfalls in Form eines Salzes mit einer anorganischen oder organischen Säure,
- Acylamino: -NH-COR, wobei R eine geradkettige, verzweigte oder cyclische, gesättigte oder ungesättigte C₁₋₁₀-Alkylgruppe bedeutet,
- Carboxy,
- C₁₋₁₀-Ester,
als Antioxidationsmittel.

12. Verwendung nach Anspruch 11 in Zusammensetzungen für die Haarbehandlung, wie Haarwaschmitteln, Lotionen, Gelen, Emulsionen, Haarlacken, Zusammensetzungen, die ausgespült werden, Zusammensetzungen, die vor oder nach der Haarwäsche, vor oder nach einer Färbung oder Entfärbung, vor, während oder nach einer permanenten Verformung oder Entkräuselung aufgetragen werden, Lotionen oder Gelen für die Frisurgestaltung oder zur Behandlung, Lotionen oder Gelen zum Fönen oder für Wasserwellen, Zusammensetzungen für permanente Verformungen oder Entkräuselungen, Zusammensetzungen zum Färben oder Entfärben der Haare; in Produkten zur Pflege der Haut oder zum Schminken, wie Produkten zum Schminken der Wimpern, der Augenbrauen oder der Haut, beispielsweise Cremes zur Behandlung der Epidermis, Make-up, Lippenstiften, Lidschatten, Wangenrouge, Mascara oder Lidstrichstiften, die auch als "Eyeliner" bezeichnet werden, Nagellacken, in Lotionen, Milchen und Cremes zur Pflege oder zur Reinigung der Haut.

13. Verwendung eines Polymers, das unter den hochverzweigten Polymeren und Dendrimeren ausgewählt ist, die funktionelle Gruppen der Formel (I) enthalten: 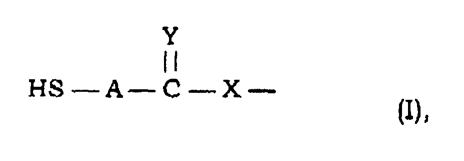 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino: -NH₂, gegebenenfalls in Form eines Salzes mit einer anorganischen oder organischen Säure,
- Acylamino: -NH-COR, wobei R eine geradkettige, verzweigte oder cyclische, gesättigte oder ungesättigte C₁₋₁₀-Alkylgruppe bedeutet,
- Carboxy,
- C₁₋₁₀-Ester,
und das auf einen Träger aufgebracht wird, zur Bildung eines Films, der aus der Bildung von intermolekularen Disulfidbrücken resultiert, die so zu einem Polymer des hochverzweigten Polymers oder Dendrimers führen.

14. Verwendung nach einem der Ansprüche 8 bis 13, wobei:
A = -CH₂-CH(CO₂H)-NH- und Y = O oder
A = -(CH₂)₂-(CH₃CONH)CH- und Y = O oder
A = -(CH₂)₃- und Y = O oder
A = -(CH₂)₃ und Y = NH.

15. Verwendung nach einem der Ansprüche 8 bis 14, wobei X ein Sauerstoffatom oder eine Gruppe -NR'- bedeutet, wobei R' unter einem Wasserstoffatom; einer geradkettigen oder verzweigten, gesättigten oder ungesättigten C₁₋₆-Alkylgruppe; einer geradkettigen oder verzweigen, gesättigten oder ungesättigten Mono- oder Polyhydroxyalkylgruppe mit 1 bis 6 Kohlenstoffatomen; einer C₁₋₆-Aminoalyklgruppe oder einer Polyalkylenimingruppe ausgewählt ist.

16. Verwendung nach einem der Ansprüche 8 bis 15, wobei das Polymer aus der Gruppe der Polyamide, aus der Gruppe der Polyalkylenpolyamine, aus der Gruppe der Polyester oder der Gruppe der Dendrimere stammt.

17. Verwendung nach einem der Ansprüche 8 bis 16, wobei das Polymer ein Polyethylenimin ist.

18. Polymer, das unter den hochverzweigten Polymeren und Dendrimeren ausgewählt ist, **dadurch gekennzeichnet, dass** es funktionelle Gruppen der Formel (I) enthält: 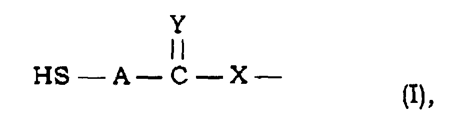 worin bedeuten:
Y ein Sauerstoffatom oder die Gruppe NH,
X eine nucleophile Gruppe, und
A eine geradkettige, verzweigte oder cyclische, gesättigte C₁₋₁₂-Alkandiylgruppe, die gegebenenfalls durch ein oder mehrere Heteroatome, wie O oder N, unterbrochen ist; wobei die Alkandiylgruppe mit einer Gruppe substituiert ist, die ausgewählt ist unter:
- Amino (-NH₂), gegebenenfalls in Form eines Salzes mit einer anorganischen oder organischen Säure,
- Acylamino (-NH-COR), wobei R eine geradkettige, verzweigte oder cyclische, gesättigte oder ungesättigte C₁₋₁₀-Alkylgruppe bedeutet,
- Carboxy,
- C₁₋₁₀-Ester.

19. Polymer nach Anspruch 18, **dadurch gekennzeichnet, dass**:
A = -CH₂-CH(CO₂H)-NH- und Y = O, oder
A = -(CH₂)₂-(CH₃CONH)CH- und Y = O.

20. Polymer nach einem der Ansprüche 18 bis 19, **dadurch gekennzeichnet, dass** X ein Sauerstoffatom oder eine Gruppe -NR'- bedeutet, wobei R' unter einem Wasserstoffatom; einer geradkettigen oder verzweigten, gesättigten oder ungesättigten C₁₋₆-Alkylgruppe; einer geradkettigen oder verzweigten, gesättigten oder ungesättigten Mono- oder Polyhydroxyalkylgruppe mit 1 bis 6 Kohlenstoffatomen; einer C₁₋₆-Aminoalkylgruppe oder einer Polyalkylenimingruppe ausgewählt ist.

21. Polymer nach einem der Ansprüche 18 bis 20, **dadurch gekennzeichnet, dass** es aus der Gruppe der Polyamide, aus der Gruppe der Polyalkylenpolyamine, aus der Gruppe der Polyester oder aus der Gruppe der Dendrimere stammt.

22. Polymer nach Anspruch 21, **dadurch gekennzeichnet, dass** es ein Polyethylenimin ist.

## Claims

1. Composition, **characterized in that** it comprises, in a cosmetically or dermatologically acceptable support, at least one polymer chosen from hyperbranched polymers and dendrimers, comprising functional groups corresponding to formula (I): 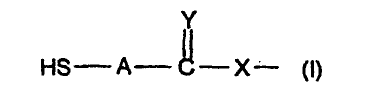 in which:
Y represents an oxygen atom or an NH group,
X represents a nucleophilic group, and
A represents a linear, branched or cyclic, saturated C₁-C₁₂ alkanediyl group; optionally interrupted with one or more hetero atoms, for instance O or N; the said alkanediyl group being substituted with a function chosen from:
- amino: -NH₂, optionally in the form of a mineral or organic acid salt,
- acylamino: -NH-COR, in which R represents a linear, branched or cyclic, saturated or unsaturated C₁-C₁₀ alkyl group,
- carboxylic acid,
- C₁-C₁₀ ester.

2. Composition, **characterized in that** it comprises, in a cosmetically or dermatologically acceptable support, at least one polymer chosen from hyperbranched polymers and dendrimers, comprising functional groups corresponding to formla (I) : 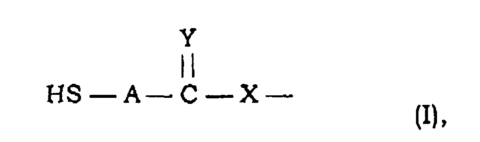 in which:
Y represents an oxygen atom or an NH group,
X represents a nucleophilic group, and
A represents a linear, branched or cyclic, saturated C₁-C₁₂ alkanediyl group; optionally interrupted with one or more hetero atoms, for instance O or N; optionally substituted with a function chosen from:
- amino: -NH₂, optionally in the form of a mineral or organic acid salt,
- acylamino: -NH-COR, in which R represents a linear, branched or cyclic, saturated or unsaturated C₁-C₁₀ alkyl group,
- carboxylic acid,
- C₁-C₁₀ ester.
combined with a compound chosen from thioglycolic acid, thiolactic acid and cysteine.

3. Composition according to either of the preceding claims, in which X represents an oxygen atom or a group -NR'- in which R' is chosen from a hydrogen atom; a linear or branched, saturated or unsaturated C₁-C₆ alkyl group; a linear or branched, saturated or unsaturated C₁-C₆ monohydroxyalkyl or polyhydroxyalkyl group; a C₁-C₆ aminoalkyl group or a polyalkyleneimine group.

4. Composition according to one of the preceding claims, in which the polymer belongs to the polyamide family, to the polyalkylenepolyamine family, to the polyester family or to the dendrimer family.

5. Composition according to one of the preceding claims, in which the polymer is a polyethyleneimine.

6. Composition according to one of the preceding claims, in which Y = O and A = -CH₂-CH(CO₂H)-NH- or -(CH₂)₂-(CH₃CONH)CH-.

7. Composition according to Claim 2, in which A = -(CH₂)₃- and Y = O or NH.

8. Use of a composition comprising, in a cosmetically or dermatologically acceptable support, at least one polymer chosen from hyperbranched polymers and dendrimers, comprising functional groups corresponding to formula (I) : 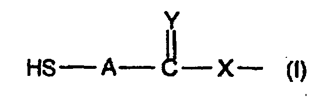 in which:
Y represents an oxygen atom or an NH group,
X represents a nucleophilic group, and
A represents a linear, branched or cyclic, saturated C₁-C₁₂ alkanediyl group; optionally interrupted with one or more hetero atoms, for instance O or N; optionally substituted with a function chosen from:
- amino: -NH₂, optionally in the form of a mineral or organic acid salt,
- acylamino: -NH-COR, in which R represents a linear, branched or cyclic, saturated or unsaturated C₁-C₁₀ alkyl group,
- carboxylic acid,
- C₁-C₁₀ ester,
to treat the nails and the hair.

9. Use of a polymer chosen from hyperbranched polymers and dendrimers, comprising functional groups corresponding to formula (I): 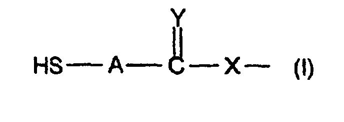 in which:
Y represents an oxygen atom or an NH group,
X represents a nucleophilic group, and
A represents a linear, branched or cyclic, saturated C₁-C₁₂ alkanediyl group; optionally interrupted with one or more hetero atoms, for instance O or N; optionally substituted with a function chosen from:
- amino: -NH₂, optionally in the form of a mineral or organic acid salt,
- acylamino: -NH-COR, in which R represents a linear, branched or cyclic, saturated or unsaturated C₁-C₁₀ alkyl group,
- carboxylic acid,
- C₁-C₁₀ ester,
as a reducing agent.

10. Use according to Claim 9, in permanent-waving compositions.

11. Use of a polymer chosen from hyperbranched polymers and dendrimers, comprising functional groups corresponding to formula (I): 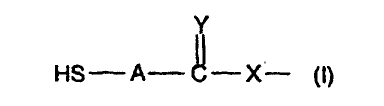 in which:
Y represents an oxygen atom or an NH group,
X represents a nucleophilic group, and
A represents a linear, branched or cyclic, saturated C₁-C₁₂ alkanediyl group; optionally interrupted with one or more hetero atoms, for instance O or N; optionally substituted with a function chosen from:
- amino: -NH₂, optionally in the form of a mineral or organic acid salt,
- acylamino: -NH-COR, in which R represents a linear, branched or cyclic, saturated or unsaturated C₁-C₁₀ alkyl group,
- carboxylic acid,
- C₁-C₁₀ ester,
as an antioxidant.

12. Use according to Claim 11, in hair compositions, as hair shampoos, lotions, gels, emulsions or lacquers, rinse-out compositions, to be appllied before or after shampooing, before or after dyeing or bleaching, before, during or after permanent-waving or relaxing the hair, styling or treatment lotions or gels, blow-drying or hairsetting lotions or gels, compositions for permanent-waving, relaxing, dyeing or bleaching the hair; in skincare products or makeup products, for instance makeup products for the eyelashes, the eyebrows or the skin, such as an epidermal treatment cream, a foundation, a tube of lipstick, an eyeshadow, a makeup rouge, a mascara, an eyeliner or a nail varnish, and in skincare or skin-cleansing lotions, milks and creams.

13. Use of a polymer chosen from hyperbranched polymers and dendrimers, comprising functional groups corresponding to formula (I): 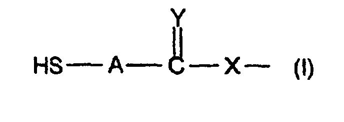 in which:
Y represents an oxygen atom or an NH group,
X represents a nucleophilic group, and
A represents a linear, branched or cyclic, saturated C₁-C₁₂ alkanediyl group; optionally interrupted with one or more hetero atoms, for instance O or N; optionally substituted with a function chosen from:
- amino: -NH₂, optionally in the form of a mineral or organic acid salt,
- acylamino: -NH-COR, in which R represents a linear, branched or cyclic, saturated or unsaturated C₁-C₁₀ alkyl group,
- carboxylic acid,
- C₁-C₁₀ ester,
applied to a support, to form a film resulting from the formation of intermolecular disulphide bridges, thus leading to a polymer of the said hyperbranched polymer or dendrimer.

14. Use according to one of Claims 8 to 13, in which:
A = -CH₂-CH(CO₂H)-NH- and Y = O, or
A = -(CH₂)₂-(CH₃CONH)CH- and Y = O, or
A = -(CH₂)₃- and Y = O, or
A = -(CH₂)₃- and Y = NH.

15. Use according to one of Claims 8 to 14, in which X represents an oxygen atom or a group -NR'- in which R' is chosen from a hydrogen atom; a linear or branched, saturated or unsaturated C₁-C₆ alkyl group; a linear or branched, saturated or unsaturated C₁-C₆ monohydroxyalkyl or polyhydroxyalkyl group; a C₁-C₆ aminoalkyl group or a polyalkyleneimine group.

16. Use according to one of Claims 8 to 15, in which the polymer belongs to the polyamide family, to the polyalkylenepolyamine family, to the polyester family or to the dendrimer family.

17. Use according to one of Claims 8 to 16, in which the polymer is a polyethyleneimine.

18. Polymer chosen from hyperbranched polymers and dendrimers, **characterized in that** it comprises functional groups corresponding to formula (I): 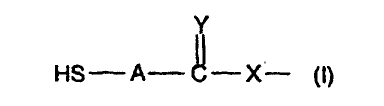 in which:
Y represents an oxygen atom or an NH group,
X represents a nucleophilic group, and
A represents a linear, branched or cyclic, saturated C₁-C₁₂ alkanediyl group, optionally interrupted with one or more hetero atoms, for instance O or N, the said alkanediyl group being substituted with a function chosen from:
- amino (-NH₂), optionally in the form of a mineral or organic acid salt,
- acylamino (-NH-COR), in which R represents a linear, branched or cyclic, saturated or unsaturated C₁-C₁₀ alkyl group,
- carboxylic acid,
- C₁-C₁₀ ester.

19. Polymer according to Claim 18, **characterized in that**:
A = -CH₂-CH(CO₂H)-NH- and Y = O, or
A = -(CH₂)₂-(CH₃CONH)CH- and Y = O.

20. Polymer according to either of Claims 18 and 19, **characterized in that** X represents an oxygen atom or a group -NR'- in which R' is chosen from a hydrogen atom; a linear or branched, saturated or unsaturated C₁-C₆ alkyl group; a linear or branched, saturated or unsaturated C₁-C₆ monohydroxyalkyl or polyhydroxyalkyl group; a C₁-C₆ aminoalkyl group or a polyalkyleneimine group.

21. Polymer according to any one of Claims 18 to 20, **characterized in that** it belongs to the polyamide family, to the polyalkylenepolyamine family, to the polyester family or to the dendrimer family.

22. Polymer according to Claim 21, **characterized in that** it is a polyethyleneimine.
